# EUROPEAN PATENT APPLICATION

(11) **EP 3 404 007 A1**
(43) Date of publication of application: **21.11.2018**
(21) Application number: 18382332.7
(22) Date of filing: 15.05.2018
(51) Int. Cl.: C07C 29/14, C07C 29/143, C07B 35/02, C07B 41/02, C07C 5/02, C07C 17/00, C07C 33/18, C07C 31/00

(54) **PROCEDURE FOR THE ACTIVATION OF ORGANIC CHLORIDE COMPOUNDS AND A CATALYTIC COMPOSITION USED IN THE PROCESS**

(30) Priority: 16.05.2017 WO PCT/ES2017/070314
(71) Applicant: Fundació Institut Català d'investigació Quimica, 43007 Tarragona (ES); Institució Catalana De Recerca I Estudis Avançats (ICREA), 08010 Barcelona (ES)
(72) Inventor: Lloret-Fillol, Julio, 46740 Carcaixent (ES); Casitas Montero, Alicia, 17006 Girona (ES); Claros Casielles, Miguel, 33402 Aviles (ES); Ungeheuer, Felix, 61118 Bad Vilbel (DE); Aragón Artigas, Jordi, 08015 Barcelona (ES)

(57) **Abstract**

The present invention relates to a composition comprising a catalyst system consisting of a cobalt(II) or nickel (II) nitrogenated complex and a copper complex; it also refers to the use of this composition as catalyst, particularly as a photocatalyst, and to a process for the activation of carbon-chlorine bonds mediated by a cobalt (II) or nickel (II) containing catalyst combined with a reducing agent.

## Description

This application claims the benefit of the PCT Patent Application PCT/ES2017/070314 filed on May 16th, 2017.

The present invention relates to a process for the activation of chlorinated saturated, unsaturated or aromatic hydrocarbons leading to the substitution of one or more chlorine atoms by hydrogen, carbon or boron atoms, and a catalytic composition used in such process.

### BACKGROUND ART

Organic halides are highly useful building blocks in the chemical industry. In particular, carbon halides bonds are less prompt to be cleaved as the electronegativity of the halide increases. Consequently, this has lead to the development of numerous procedures for the cleavage of carbon-iodine and carbon-bromine bonds. On the other hand, the cleavage of carbon-chlorine bonds is still particularly challenging and few synthetic procedures have been reported for the substitution of carbon-chlorine bonds, especially for saturated chlorinated hydrocarbons. Besides being typically easier and cheaper to prepare than brominated and iodinated compounds, chlorinated compounds are usually poorly reactive and the substitution of the chlorine atom for a weak nucleophilic atom still remains a difficult reaction. Chloroalkanes are predominant patterns in both commercially available building blocks and biologically active molecules, including pharmaceutical and agrochemical compounds. On the other hand, the inertness of chloroalkanes have precluded them as prevailing electrophilic coupling partners in transition-metal catalyzed cross-coupling reactions. Furthermore, the use of light as driving force for chemical reactions has attracted much attention in organic chemistry and materials science. The use of light allows switching a reaction on and off with time and spatial control of the reaction being carried out in mild conditions and with high selectivity. Consequently, photochemical transformations of organic halides, and more particularly of organic chlorides, is an attractive and sustainable way of preparing organic compounds. The large negative reduction potential of alkyl chlorides however discards them as suitable precursors for forming carbon-centered radicals by single electron transfer (SET) reactions from photoredox catalysts (such as Ru and Ir polypyridyl complexes). The current state-of-the-art bimetallic catalyst formed by iridium polypyridyl compounds in combination with nickel bipyridine complexes have shown a remarkable performance in C-C bond-forming reactions (Twilton et al. Nature Reviews Chemistry 2017, 1, article 52). Nevertheless, this widely employed bimetallic catalytic system is not active for the cleavage of strong Csp³-Cl bonds under visible-light irradiation.

Barriault and co-workers reported in 2013 (Angew. Chem. Int. Ed. 2013, 52, 13342 -13345) a gold-catalyzed photocatalytic procedure for the intramolecular cyclization of alkyl bromides having a carbon-carbon double bond at a remote position. The same catalyst system is reported to allow for dehalogenation of aryl bromides. According to the authors, a radical mechanism is involved in the procedure. Authors also report the intermolecular reaction of an activated chloroalkane (N-chloromethylphthalimide) with a diene compound. Authors are however silent about the effectiveness of the catalytic system when unactivated chloroalkanes are used.

Ghosh and co-workers also reported in 2014 a procedure for the dehalogenation and cross-coupling reactions of aryl halides, including aryl chlorides, using a polyaromatic compound able to absorb visible light combined with a tertiary amine as electron donor to activate the reaction substrate (Science 2014, 346, 725-728). Although being reactive with the reported system, electron poor aryl chlorides typically require high loading of photosensitizer (up to 10 mol%) and longer reaction times. The authors however do not report the activation of alkyl halides with the photocatalytic system.

On a different approach, Lee and Stephenson separately reported the photocatalytic activation of aryl and alkyl iodides using iridium based photosensitizers combined with tertiary amines as electron donors. Such systems allow performing dehydrogenation of aryl iodides or intermolecular cyclization when the reaction substrate comprises in its molecular formula an unsaturated carbon-carbon bond in such a way that 5 or 6-membered ring is formed upon the cyclization (Stephenson and co-workers Chem.Comm. 2010, 46, 4985-4987; Lee and co-workers, Angew. Chem. Int. Ed. 2012, 51, 12303-12306). Later on, Martin and co-workers also employed a similar system to functionalize alkyl iodides (ACS Catalysis, 2017, 7, 409-412). Authors are however silent about the reaction with chlorinated substrates. Based on a similar catalyst system, Stephenson and co-workers (Nature Chemistry 2012, 4, 854-859) showed the use of iridium phsotosensitizer combined with tertiary amines and eventually formic acid as hydrogen donor promotes the dehalogenation of alkenyl iodides. Likewise, under these reaction conditions, intramolecular cyclization of alkyl iodides bearing an unsaturation at a remote distance allowed the formation of 5 to 6-membered ring products. According to the authors, aryl chlorides remain untouched by the reported catalytic system.

Thus, from what is known in the state-of-the-art, it is derived that the development of an improved process for the dehydrogenation and cross-coupling of organic chlorides taking place in mild and robust conditions is still of great interest.

### SUMMARY OF THE INVENTION

Inventors have developed an efficient, general, selective and robust process in order to activate carbon-chlorine bonds with a dual catalytic system involving a cobalt (II) or nickel (II) compound that acts as a catalyst and a reducing agent, such as a redox photosensitizer with a reduction potential that is adequate to reduce the catalyst, which promotes the formation of carbon-carbon bonds, carbon-hydrogen bonds or carbon-boron bonds from carbon-chloride bonds.

The process is advantageous since it allows activating organic chloride compounds that cannot be activated otherwise and uses a catalyst system that is robust and based on abundant metal atoms.

Therefore one aspect of the invention relates to a composition comprising a catalyst system consisting of: a compound of formula

[Cu(L₁)(L₂)]Z₁,

wherein:
Cu is in the oxidation state (+1);
Z₁ is an anion
L₁ is a bidentate phosphine ligand attached to the Cu atom through the P atoms
L₂ is a bidentate nitrogen ligand attached to the Cu atom through two N atoms consisting of a ring system comprising from 2 to 7 rings, wherein: the rings have from 5 to 6 members selected from the group consisting of C, CH, N, and NH; at least two members being N or NH;
the N atoms attached to the copper atom are separated by no more than three ring members and are members of different rings;
the rings are unsaturated or aromatic;
the rings are fused or interconnected;
the rings are optionally substituted with one or more radicals selected from the group consisting of: (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro;
and a cobalt (II) or nickel (II) compound selected from the compounds of formula [Co(L₃)X]⁺X'⁻, [Co(L₃)XX'], [Ni(L₃)X]⁺X'⁻ and [Ni(L₃)XX'] wherein:
   X and X' are anions equal or different;
   L₃ is a tetradentate or pentadentate nitrogenated ligand attached to the Co or Ni atom through four or five N atoms that is selected from the compounds of formula (Ia), (Ib), (Ic), (Id) and (Ie) 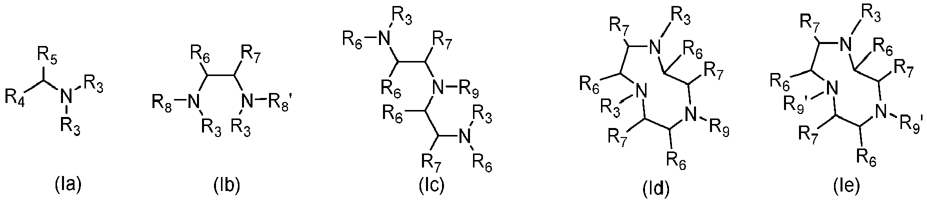 wherein each R₃ is a radical of formula (II) 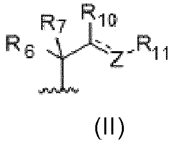
      wherein Z is N or NH;
      the dashed bond represents a single C-Z bond or a double C=Z bond; and R₁₀ and R₁₁, together with the atoms to which they are attached, form a ring system comprising from one to three unsaturated or aromatic 5 to 6-membered rings, said rings being fused or interconnected, the members of the ring being selected from C, CH, N and NH, the rings being further optionally susbtituted with one or more radicals selected from the group consisting of (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro; R₄ and R₅ are each independently selected from the group consisting of: hydrogen, (C₁-C₆)alkyl, (C₁-C₆)haloalkyl, and a radical of formula -C(R₁₀)(ZR₁₁); being at least one of R₄ and R₅ a radical of formula -C(R₁₀)(ZR₁₁);
      R₆ and R₇ are each independently selected from the group consisting of: hydrogen, (C₁-C₆)alkyl, halo, (C₁-C₆)haloalkyl, and (C₁-C₆)alkyloxy; or, alternatively,
      R₆ and R₇, together with the carbon atoms to which they are attached form a (C₃-C₈)cycloalkyl optionally substituted with one or more radicals selected from the group consisting of: (C₁-C₆)alkyl, halo, (C₁-C₆)haloalkyl, and (C₁-C₆)alkyloxy;
      R₈ is a radical selected from the group consisting of hydrogen, (C₁-C₆)alkyl, and (C₁-C₆)haloalkyl;
      R_{8'} is a radical selected from the group consisting of hydrogen, (C₁-C₆)alkyl, (C₁-C₆)haloalkyl and a radical of formula (II);
      or, alternatively,
      one or two of the pairs of R₆ and R₈ and R₇ and R₈' together with the carbon atoms to which they are attached form a (C₃-C₈)Cycloalkyl optionally substituted with one or more radicals selected from the group consisting of (C₁-C₆)alkyl, halo, (C₁-C₆)haloalkyl, and (C₁-C₆)alkyloxy;
      Rg and Rg' are each independently selected from the group consisting of hydrogen, (C₁-C₆)alkyl, a radical of formula R₁₂O(C=O)-, R₁₂(C=O)-, R₁₂(S=O)- and R₁₂(SO₂)-wherein R₁₂ is selected from the group consisting of (C₁-C₆)alkyl, (C₁-C₆)haloalkyl, benzyl and phenyl optionally substituted with (C₁-C₆)alkyl and (C₁-C₆)haloalkyl; and wherein:
         when the cobalt (II) compound is a compound of formula [Co(L₃)X]⁺X'⁻, L₃ is a pentadentate nitrogenated ligand; and
         when the cobalt (II) compound is a compound of formula [Co(L₃)XX'], L₃ is a tetradentate nitrogenated ligand;
      when the nickel (II) compound is a compound of formula [Ni(L₃)X]⁺X'⁻, L₃ is a pentadentate nitrogenated ligand; and
      when the nickel (II) compound is a compound of formula [Ni(L₃)XX'], L₃ is a tetradentate nitrogenated ligand.

The composition of the first aspect of the invention is useful as a photoredox catalyst. Other aspect of the invention therefore relates to the use of the composition of the first aspect as a catalyst in combination with an electron donor and a source of light. This catalyst system is able to activate carbon-chlorine bonds and promote the substitution of the chlorine atom by a carbon, hydrogen or boron atom under mild reaction conditions. The dual catalytic system can operate in aqueous mixtures and tolerates an oxygen containing atmosphere, unlike most transition metal catalysts used in cross-coupling reactions.

A third aspect of the invention also refers to a process for the preparation of a compound comprising one or more moieties of formula C₁-Y from a compound comprising one or more moieties of formula C₁-Cl, the moieties of formula C₁-Cl being converted in the moieties of formula C₁-Y, wherein C₁ is a saturated carbon atom or a carbon atom comprised in a carbon-carbon double bond and Y is an atom selected from C₂, H and B, being C₂ a saturated carbon atom, said process comprising the step of contacting the compound comprising one or more moieties of formula C₁-Cl with a catalytically effective amount of a cobalt (II) or nickel (II) coordination complex with a tetradentate or pentadentate nitrogenated ligand in the presence of a reducing agent and wherein:
when Y is C₂, the moiety of formula C₁-Cl is comprised in a moiety of formula (III) and the moiety of formula C₁-C₂ is comprised in a moiety of formula (IV) 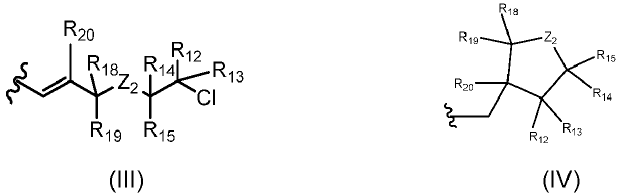 wherein Z₂ is selected from NR₁₆, CR₁₆R₁₇, S, PR₁₆ and O,
each of R₁₂, R₁₃, R₁₄, R₁₅, R₁₆, R₁₇, R₁₈, R₁₉ and R₂₀ is independently selected from the group consisting of hydrogen, (C₁-C₆)alkyl, (C₂-C₆)alkenyl, (C₂-C₆)alkynyl, (C₁-C₆)haloalkyl, (C₁-C₆)alkyloxy, (C₁-C₆)alkylamino, (C₁-C₆)alkylcarbonyl, (C₁-C₆)alkylcarbonyloxy, (C₁-C₆)alkyloxycarbonyl, (C₁-C₆)alkylcarbonylamino, (C₁-C₆)alkylaminocarbonyl, cyano and nitro; or alternatively, one or two of the pairs of R₁₃ and R₁₄, R₁₄ and R₁₆, R₁₆ and R₁₉ and R₁₉ and R₂₀, together with the carbon atoms to which they are attached, form a ring system comprising from 1 to 3 saturated, unsaturated or aromatic rings, where chemically possible, each ring comprising from 3 to 8 members selected from the group consisting of C, CH, CH₂, O, N, CO, CS and S, the rings being further optionally substituted at any available position with one or more groups selected from the group consisting of (C₁-C₆)alkyl, (C₂-C₆)alkenyl, (C₂-C₆)alkynyl, (C₁-C₆)haloalkyl, (C₁-C₆)alkyloxy, (C₁-C₆)alkylamino, (C₁-C₆)alkylcarbonyl, (C₁-C₆)alkylcarbonyloxy, (C₁-C₆)alkyloxycarbonyl, (C₁-C₆)alkylcarbonylamino, (C₁-C₆)alkylaminocarbonyl, cyano and nitro;
R₁₆ and R₁₇ are each independently selected from the group consisting of hydrogen, (C₁-C₆)alkyl, (C₂-C₆)alkenyl, (C₂-C₆)alkynyl, (C₁-C₆)haloalkyl, (and (C₁-C6)alkylcarbonyl; or, alternatively, the moieties of formula CR₁₄R₁₅ and CR₁₆R₁₇, CR₁₈R₁₉ further optionally form a carbonyl group;
   or, alternatively,
when Y is B, C₁ is comprised in a carbon-carbon double bond and the process is further carried out and in the presence of a compound of formula [B(OR₂₁)(OR₂₂)]₂ wherein each of R₂₁ and R₂₂ is independently selected from (C₁-C₆)alkyl, (C₂-C₆)alkenyl, (C₂-C₆)alkynyl, and (C₁-C₆)haloalkyl; or, alternatively, R₂₁ and R₂₂, together with the oxygen atoms to which they are attached, said oxygen atoms being bridged by a boron atom, form a 5 to 6-membered saturated
carbocyclic ring optionally substituted at any available position with one or more (C₁-C₆)alkyl groups.

### DETAILED DESCRIPTION OF THE INVENTION

In the present description:
The term "halogen" or "halo" refers to iodine, bromine, chlorine and fluorine.

The term "alkyl", alone or in combination, refers to a straight-chain or branched-chain saturated hydrocarbon group having the indicated number of carbon atoms. For example, C₁₋₆ alkyl refers to an alkyl group having from one to six carbon atoms with the remaining valences occupied by hydrogen atoms. Examples of straight-chain and branched C₁₋₆ alkyl group include but are not limited to, methyl, ethyl, propyl, isopropyl, butyl, isobutyl, tert-butyl, the isomeric pentyls, the isomeric hexyls and the like.

The term "haloalkyl" refers to radicals wherein one or more of the alkyl carbon atoms are substituted with one or more halogen atoms.

The term "alkenyl" embraces linear or branched radicals having the indicated number of carbon atoms and containing at least one double bond.

The term "alkynyl" embraces linear or branched radicals having the indicated number of carbon atoms and containing at least one carbon-carbon triple bond. The term "alkyloxy" means an alkyl substituent attached to the remainder of a molecule via oxygen, in which the term "alkyl" has the previously given definition. Examples of alkyloxy group include, but are not limited to, methoxy, ethoxy, n-propoxy, isopropoxy, n-butoxy, isobutoxy, sec-butoxy and tert-butoxy.

The term "alkylcarbonyloxy" includes alkyl as defined above bonded to carbonyl bonded to oxygen (-O-C(O)-alkyl). Examples of alkylcarbonyloxy group include, but are not limited to, acetate, propionate and t-butylcarbonyloxy.

The term "alkyloxycarbonyl" means a radical containing an alkyloxy radical, as defined above, attached via the oxygen atom to a carbonyl radical (-C(O)-O-alkyl). Examples of such radicals include, but are not limited to, substituted or unsubstituted methoxycarbonyl, ethoxycarbonyl, propoxycarbonyl, butoxycarbonyl and hexyloxycarbonyl.
The term "aromatic ring system" means an aromatic hydrocarbon group which can be a single ring or multiple rings which are fused together or linked covalently, and which optionally carries one or more substituents.
The term "heteroaromatic ring sytem", alone or in combination, refers to an aromatic 5- to 10-membered heterocycle which contains one or more, preferably one or two hetero atoms selected from nitrogen, oxygen, and sulfur. The heteroaryl may be substituted on one or more carbon atoms as defined herein, for example with substituents such as halogen, alkyl, alkoxy, cyano, haloalkyl.
The term "cycloalkyl" refers to a saturated monocyclic or bicyclic cycloalkyl group. Examples of cycloalkyl groups include cyclopropyl, cyclobutyl, cyclopentyl.
The term "reducing agent" refers to a chemical species or system able to give an electron to the cobalt (II) or nickel(II) compound used in the process of the invention. Examples include photosensitizers combined with tertiary amines, electrochemical means, reduced forms of carbon dioxide (methanol, formaldehyde, formic acid, lactic acid) and metals (such as Zn or Mn).

In a particular embodiment of the first aspect of the invention, optionally in combination with one or more of the embodiments described below, the cobalt (II) or nickel (II) compound of the composition is selected from the compounds of formula [Co(L₃)X]⁺X'⁻ [Ni(L₃)X]⁺X'⁻, [Ni(L₃)XX'] and [Co(L₃)XX'] wherein X and X' are equal are selected from halide and coordinating oxoanions such as trifluoromethanesulfonate, and non-coordinating anions such as hexafluorophosphate, tetrafluoroborate and the like.

In a particular embodiment of the first aspect of the invention, optionally in combination with one or more of the embodiments described above or below, the cobalt (II) or nickel (II) compound of the composition is selected from the compounds of formula [Co(L₃)X]⁺X'⁻, [Ni(L₃)X]⁺X'⁻, [Ni(L₃)XX'] and [Co(L₃)XX'] wherein:
X and X' are equal are selected from chloride and trifluoromethanesulfonate;
L₃ is a tetradentate or pentadentate nitrogenated ligand attached to the Co or Ni atom through four or five N atoms that is selected from the compounds of formula (Ia), (Ib), (Ic), (Id) and (Ie) as defined above wherein each R₃ is a radical of formula (II) as defined above wherein:
   Z is N;
   R₁₀ and R₁₁, together with the atoms to which they are attached, form a pyridyl ring optionally substituted at any available position with one or more groups selected from the group consisting of (C₁-C₆)alkyloxy and (C₁-C₆)alkyloxycarbonyl;
   R₆ and R₇ are each hydrogen;
   R₄ and R₅ are each selected from the group consisting of hydrogen, 2-pyridyl and 6-(2-pyridyl)-2-pyridyl;
   R₈ is a radical selected from the group consisting of hydrogen and methyl;
   R_{8'} is a radical selected from the group consisting of hydrogen, methyl and a radical of formula (II) wherein R₆ and R₇ are hydrogen, Z is N and R₁₀ and R₁₁, together with the atoms to which they are attached form a pyridyl ring; or, alternatively,
   the two of the pairs of R₆ and R₈ and R₇ and R_{8'} together with the carbon atoms to which they are attached form a cyclopentyl;
   R₉ is a radical of formula R₁₂(SO₂)- wherein R₁₂ is a tolyl group; and
   R₉' is a methyl group.

In a more particular embodiment of the first aspect of the invention, optionally in combination with one or more of the embodiments described above or below, the cobalt (II) or nickel (II) compound of the composition is selected from the compounds of formula [Co(L₃)X]⁺X'⁻, [Ni(L₃)X]⁺X'⁻, [Ni(L₃)XX'] and [Co(L₃)XX'] wherein L₃ is a compound of formula (Ia) as defined above wherein:
R₃ is a radical of formula (II) wherein Z is N, R₆ and R₇ are hydrogen and R₁₀ and
R₁₁, together with the atoms to which they are attached, form a pyridyl ring;
R₄ and R₅ are independently selected from the group consisting of hydrogen, 2-pyridyl, and 6-(2-pyridyl)-2-pyridyl.

In a more particular embodiment of the first aspect of the invention, optionally in combination with one or more of the embodiments described above or below, the cobalt (II) or nickel (II) compound of the composition is selected from the compounds of formula [Co(L₃)X]⁺X'⁻, [Ni(L₃)X]⁺X'⁻, [Ni(L₃)XX'] and [Co(L₃)XX'] wherein L₃ is a compound of formula (Ib) as defined above wherein:
R₃ is a radical of formula (II) wherein Z is N, R₆ and R₇ are hydrogen and R₁₀ and
R₁₁, together with the atoms to which they are attached, form a pyridyl ring;
R₆ and R₇, together with the carbon atoms to which they are attached form a cyclohexyl group;
R₈ is a radical selected from the group consisting of hydrogen and methyl;
R_{8'} is a radical selected from the group consisting of hydrogen, methyl and a radical of formula (II) wherein R₆ and R₇ are hydrogen, Z is N and R₁₀ and R₁₁, together with the atoms to which they are attached form a pyridyl ring;
or, alternatively,
the two of the pairs of R₆ and R₈ and R₇ and R₈' together with the carbon atoms to which they are attached form a cyclopentyl.

In a more particular embodiment of the first aspect of the invention, optionally in combination with one or more of the embodiments described above or below, the cobalt (II) or nickel (II) compound of the composition is selected from the compounds of formula [Co(L₃)X]⁺X'⁻, [Ni(L₃)X]⁺X'⁻, [Ni(L₃)XX'] and [Co(L₃)XX'] wherein L₃ is selected from the compounds of formula (Id) as defined above wherein:
R₃ is a radical of formula (II) wherein Z is N, R₆ and R₇ are hydrogen and R₁₀ and
R₁₁, together with the atoms to which they are attached, form a pyridyl ring;
R₆ and R₇ are hydrogen and Rg is a radical of formula R₁₂(SO₂)- wherein R₁₂ is a tolyl group.

In a more particular embodiment of the first aspect of the invention, optionally in combination with one or more of the embodiments described above or below, the cobalt (II) or nickel (II) compound of the composition is selected from the compounds of formula [Co(L₃)X]⁺X'⁻, [Ni(L₃)X]⁺X'⁻, [Ni(L₃)XX'] and [Co(L₃)XX'] wherein L₃ is selected from the compounds of formula (le) as defined above wherein:
R₃ is a radical of formula (II) wherein Z is N, R₆ and R₇ are hydrogen and R₁₀ and
R₁₁, together with the atoms to which they are attached, form a pyridyl ring;
R₆ and R₇ are hydrogen and Rg' is a methyl group.

In a more particular embodiment of the first aspect of the invention, optionally in combination with one or more of the embodiments described above or below, the cobalt (II) or nickel (II) compound is selected from: [M(X)(Py₂^{Ts}tacn)](X), [M(X)(Py₂^{Me}tacn)](X), [M(X)(DPA-Bpy)](X), [M(X)(N4Py)](X), [M(X)₂(PDP)], [M(X)(TPA)](X), and [M(X)₂(mcp)] 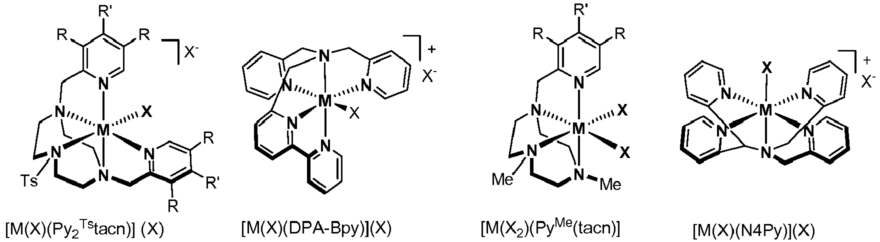 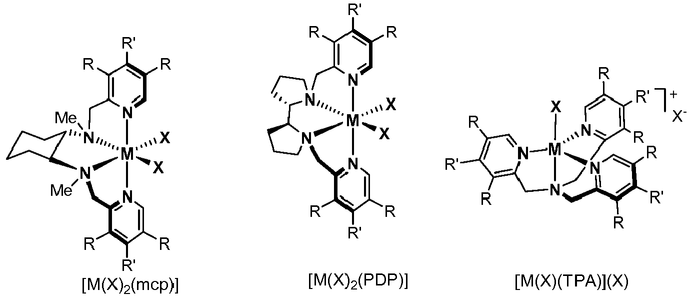 being M selected from Co and Ni and X being selected from chloride and trifluoromethanesulfonate and wherein R and R' are each a radical independently selected from the group consisting of hydrogen, (C₁-C₆)alkyl, (C₁-C₆)alkyloxy, and (C₁-C₆)alkyloxycarbonyl.

In a more particular embodiment of the first aspect of the invention, optionally in combination with one or more of the embodiments described above or below, the cobalt (II) or nickel (II) compound is [Co(OTf)(Py₂^{Ts}tacn)](OTf) or [Ni(OTf)(Py₂^{Ts}tacn)](OTf); preferably it is [Ni(OTf)(Py₂^{Ts}tacn)](OTf).

In a more particular embodiment of the first aspect of the invention, optionally in combination with one or more of the embodiments described above or below the compound of formula Cu(L₁)(L₂)]Z₁ is that wherein L₁ is a compound of formula P(R₂₁R₂₁')-Q-P(R₂₂R₂₂') wherein each of R₂₁, R₂₁' R₂₂ and R₂₂' is independently selected from the group consisting of (C₁-C₆)alkyl, (C₁-C₆)alkyloxy, and phenyl that is optionally substitued with one or more radicals selected from the group consisting of (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro;
Q is selected from the diradicals deriving from the radicals (C₂-C₆)alkyl, (C₃-C₈)cycloalkyl, and a diradical of formula (V) 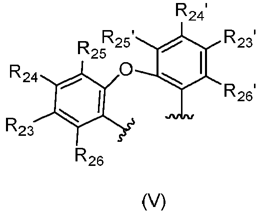 wherein:
each of R₂₃, R₂₃', R₂₄, R₂₄', R₂₅, R₂₅', R₂₆ and R₂₆' is independently selected from the group consisting of hydrogen, (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro; and
R₂₆ and R₂₆' further optionally form together a diradical of formula -(A)ₙ- wherein n is an integer from 0 to 1 and A is selected from the group consisting of CH₂, CH₂-CH₂, S, C((C₁-C₆)alkyl)₂, PR₂₁, Si((C₁-C₆)alkyl)₂, and (C₁-C₆)alkenyl.

In a more particular embodiment of the first aspect of the invention, optionally in combination with one or more of the embodiments described above or below the compound of formula [Cu(L₁)(L₂)]Z₁ is that wherein L₁ is a compound of formula P(R₂₁R₂₁')-Q-P(R₂₂R₂₂') wherein each of R₂₁, R₂₁' R₂₂ and R₂₂' is phenyl;
Q is a diradical of formula (V) as defined above wherein R₂₆ and R₂₆' form together a diradical of formula -(A)ₙ- wherein n is 1 and A is C((C₁-C₆)alkyl)₂ and wherein each of R₂₃, R₂₃', R₂₄, R₂₄', R₂₅ and R₂₅' is independently selected from the group consisting of hydrogen, (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro.

In a more particular embodiment of the first aspect of the invention, optionally in combination with one or more of the embodiments described above or below the compound of formula [Cu(L₁)(L₂)]Z₁ is that wherein L₁ is Xantphos of formula:

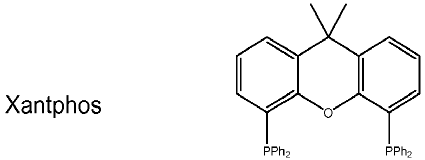

In a more particular embodiment of the first aspect of the invention, optionally in combination with one or more of the embodiments described above or below, the compound of formula [Cu(L₁)(L₂)]Z₁ is that wherein L₂ is a compound of formula (VI) 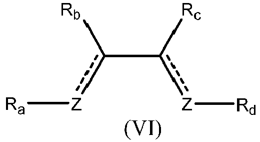 wherein:
Z is N or NH;
the dashed bond represents a single C-Z bond or a double C=Z bond;
each of the pairs Rₐ and R_{b} and R_{c} and R_{d}, together with the atoms to which they are attached, form a ring system, that is equal or different, each ring system comprising from one to four unsaturated or aromatic 5 to 6-membered rings, the rings being fused or interconnected, the members of the rings being selected from C, CH, N, NH and O and the rings being further optionally substituted with one or more radicals selected from the group consisting of (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro; and wherein the pair R_{b} and R_{c} optionally further forms a ring system comprising from one to two unsaturated or aromatic 5 to 6-membered rings, the rings being fused or interconnected, the members of the rings being selected from C, CH, N, NH and O and the rings being further optionally substituted with one or more radicals selected from the group consisting of (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro.

Preferably, optionally in combination with one or more of the embodiments described above or below, the compound of formula [Cu(L₁)(L₂)]Z₁ is one wherein L₂ is a compound of formula (VI) as defined above wherein:
Z is N;
each of the pairs Rₐ and R_{b} and R_{c} and R_{d}, together with the atoms to which they are attached, form a phenyl ring optionally substituted with one or more radicals selected from the group consisting of (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro; and wherein the pair R_{b} and R_{c}, together with the carbon atoms to which they are attached, forms a phenyl ring that is optionally substituted with one or more radicals selected from the group consisting of (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro. More preferably, the compound of formula [Cu(L₁)(L₂)]Z₁ is one wherein L₂ is bathocuproine.

In a preferred embodiment of the first aspect of the invention, optionally in combination with one or more of the embodiments described above or below, the compound of formula [Cu(L₁)(L₂)]Z₁ is one of formula:

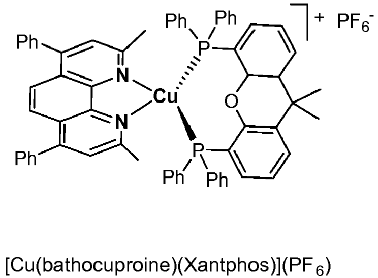

In a more particular embodiment of the first aspect of the invention, the composition comprises a catalyst composition consisting of a compound of formula [Cu(bathocuproine)(Xantphos)](PF₆) and the cobalt (II) or nickel (II) compound that is selected from: [M(X)(Py₂^{Ts}tacn)](X), [M(X)(Py₂^{Me}tacn)](X), [M(X)(DPA-Bpy)](X), [M(X)(N4Py)](X), [M(X)₂(PDP)], [M(X)(TPA)](X), and [M(X)₂(mcp)] 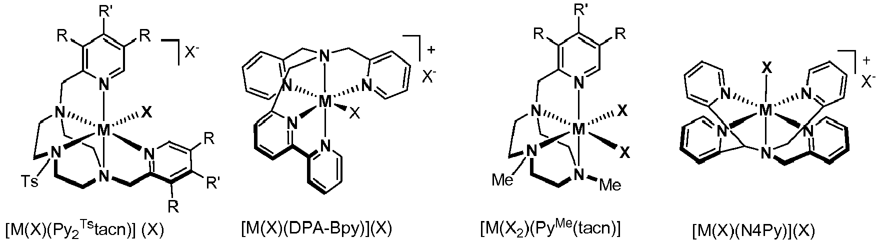 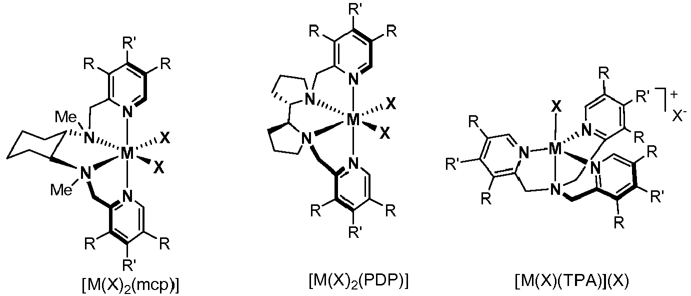 being M selected from Co and Ni and X being selected from chloride and trifluoromethanesulfonate and wherein R and R' are each a radical independently selected from the group consisting of hydrogen, (C₁-C₆)alkyl, (C₁-C₆)alkyloxy, and (C₁-C₆)alkyloxycarbonyl. Preferably, the composition comprises a catalyst composition consisting of a compound of formula [Cu(bathocuproine)(Xantphos)](PF₆) and [Ni(OTf)(Py₂^{Ts}tacn)](OTf) or [Co(OTf)(Py₂^{Ts}tacn)](OTf).

In a more particular embodiment of the first aspect of the invention, the composition comprises a catalyst composition as defined above wherein the molar ratio of the compound of formula [Cu(L₁)(L₂)]Z₁ to the cobalt (II) or nickel (II) compound is comprised from 0.2:1 to 1:1; preferably it is 0.4:1.

As mentioned above, the composition of the first aspect of the invention is useful as a catalyst in photocatalytic processes, in particular when used in combination with an electron donor and a light source. A second aspect of the invention thus relates to the use of the composition of the first aspect of the invention in combination with an electron donor and a light source.

In a more particular embodiment of the second aspect of the invention, the electron donor is selected from the group consisting of the compounds of formula NR₂₃R₂₄R₂₅ wherein each of R₂₃, R₂₄ and R₂₅ is independently a (C₁-C₆)alkyl. Even more particularly, optionally in combination with one or more of the embodiments described above or below, the electron donor is selected from triethylamine and diisopropylethylamine. Tertiary amines may be used in large excess or as part of the solvent system in the process of the invention. Alternatively, the electron donor can be selected from the reduced forms of CO₂, being such reduced form of CO₂ preferably selected from the group consisting of methanol, formaldehyde, formic acid, oxalic acid, ascorbic acid and lactic acid. The electron donor may alternatively consist of an electrode such as an electrochemical half-cell or a cathode.

In a more particular embodiment of the second aspect of the invention, optionally in combination with one or more of the embodiments described below, the light source is a source of visible light. As known in the state of the art, "visible light" refers to an irradiation in the range of wavelength from 400 to 800 nm In a more particular embodiment of the process of reduction of the invention, optionally in combination with one or more of the embodiments described below, the light source is suitable to induce a change in the electronic configuration of the compound of formula [Cu(L₁)L₂)]Z₁, promoting an electron of the copper compound to a molecular orbital of higher energetic level. More particulary, the wavelength of the light source is in the range from 400 to 500 nm. Even more particularly, optionally in combination with one or more of the embodiments described below, the wavelength of the light source is 447 nm. In a more particular embodiment of the second aspect of the invention, optionally in combination with one or more of the embodiments described below, when the compound of formula [Cu(L₁)(L₂)]Z₁ is [Cu(bathocuproine)(xanthphos)](PF₆), the source of light has a wavelength comprised from 400 to 500 nm, more preferably of 450 nm.

As described in PCT/ES2017/070314, the composition of the invention, when a cobalt (II) compound is used, is useful as a catalyst system for the reduction of ketones and alkenes. According to the third aspect of the invention, the invention relates to a process for the activation of carbon-chlorine bonds, that is carried out in the presence of a catalytically effective amount of a cobalt (II) or nickel (II) coordination complex with a tetradentate or pentadentate nitrogenated ligand in the presence of a reducing agent.

A suitable reducing agent to carry out the process of the third aspect of the invention is able to reduce the cobalt (II) or nickel (II) compound to a cobalt (I) or nickel (I) compound and is selected from the group consisting of photochemical reducing agents, electrochemical reducing agents and chemical reducing agents, and wherein when the reducing agent is a photochemical reducing agent, the process is further carried out under light irradiation.

In a more particular embodiment of the third aspect of the invention, optionally in combination with one or more of the embodiments described below, the cobalt (II) or nickel (II) coordination complex with a tetradentate or pentadentate nitrogenated ligand is as defined in the first aspect of the invention.

In a more particular embodiment of the third aspect of the invention, optionally in combination with one or more of the embodiments described below, the reducing agent is an electrochemical reducing agent, such as a cathode. In an alternative embodiment, optionally in combination with one or more of the embodiments described below, the reducing agent is a chemical reducing agent, such as zinc or manganese.

In a more particular embodiment of the third aspect of the invention, optionally in combination with one or more of the embodiments described below, the reducing agent is a photochemical reduing agent, consisting of the combination of a photosensitizer and an electron donor. In particular, the photosensitizer has an oxidation potential higher than the oxidation potential of the cobalt (II) compound or nickel (II) compound.

In a particular embodiment of the third aspect of the invention, optionally in combination with one or more of the embodiments described above or below, the photosensitizer is selected from the group consisting of chlorophylls, eosin Y, rose bengal, hexacoordinated ruthenium(II) complexes with polypyridyl ligands, hexacoordinated iridium(III) complexes with pyridyl and/or polypyridyl ligands, and a compound of formula [Cu(L₁)(L₂)]Z₁ as defined in the first aspect of the invention. More particularly, the photosensitizer is selected from the group consisting of hexacoordinated ruthenium(II) complexes with polypyridyl ligands, hexacoordinated iridium(III) complexes with pyridyl and/or polypyridyl ligands, and a compound of formula [Cu(L₁)(L₂)]Z₁ as defined in the first aspect of the invention and the electron donor is selected form the group consisting of a compound of formula NR₂₃R₂₄R₂₅ wherein each of R₂₃, R₂₄ and R₂₅ is independently a (C₁-C₆)alkyl, methanol, formaldehyde, formic acid, oxalic acid, ascorbic acid, lactic acid and an electrochemical half-cell.

In a more particular embodiment of the the third aspect of the invention, optionally in combination with one or more of the embodiments described above or below, the the photosensitizer is selected from the group consisting of hexacoordinated ruthenium(II) complexes with polypyridyl ligands, hexacoordinated iridium(III) complexes with pyridyl and/or polypyridyl ligands, and a compound of formula [Cu(L₁)(L₂)]Z₁ as defined in the first aspect of the invention.

In a more particular embodiment of the third aspect of the invention, optionally in combination with one or more of the embodiments described above or below, the the photosensitizer is selected from the group consisting of [Ru(bpy)₃](PF₆)₂, [Ir(ppy)₂(bpy)]PF₆ and a compound of formula [Cu(L₁)(L₂)]Z₁ as defined in the first aspect of the invention

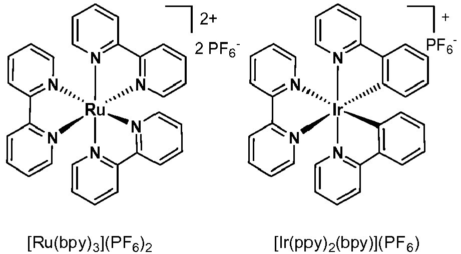

In a particular embodiment of the the third aspect of the invention, optionally in combination with one or more of the embodiments described above or below, the photosensitizer is a compound of formula [Cu(L₁)(L₂)]Z₁ as defined above. Such compound of formula [Cu(L₁)(L₂)]Z₁ is preferably a compound wherein L₁ is a diphosphine having a bite angle in the range from 95° to 125°.

More particularly, optionally in combination with one or more of the embodiments described above or below, the photosensitizer is a compound of formula [Cu(L₁)(L₂)]Z₁ as defined in the first aspect of the invention.

In a more particular embodiment of the third aspect of the invention, optionally in combination with one or more of the embodiments described above or below, the photosensitizer is a compound of formula [Cu(L₁)(L₂)]Z₁ as defined in any of the embodiments of the first aspect of the invention, the electron donor is a compound of formula NR₂₃R₂₄R₂₅ wherein each of R₂₃, R₂₄ and R₂₅ is independently a (C₁-C₆)alkyl and the cobalt (II) or nickel (II) compound is as defined in any of the embodiments of the first aspect of the invention defined above. Preferably, the photosensitizer is [Cu(bathocuproine)(xanthphos)](PF₆), the electron donor is selected from the group consisting of trimethylamine and diisopropylethylamine and the cobalt (II) or nickel (II) compound is as defined is selected from the group consisting of the compounds of formula [M(X)(Py₂^{Ts}tacn)](X), [M(X)(Py₂^{Me}tacn)](X), [M(X)(DPA-Bpy)](X), [M(X)(N4Py)](X), [M(X)₂(PDP)], [M(X)(TPA)](X), and [M(X)₂(mcp)] 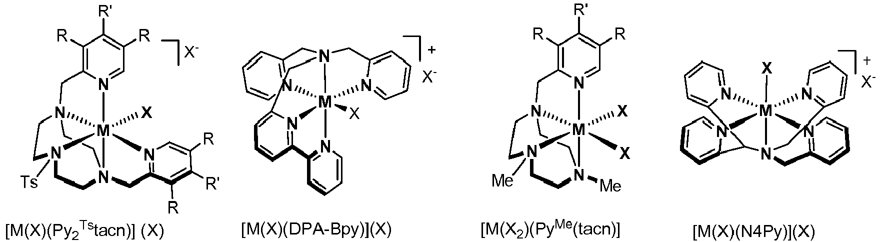 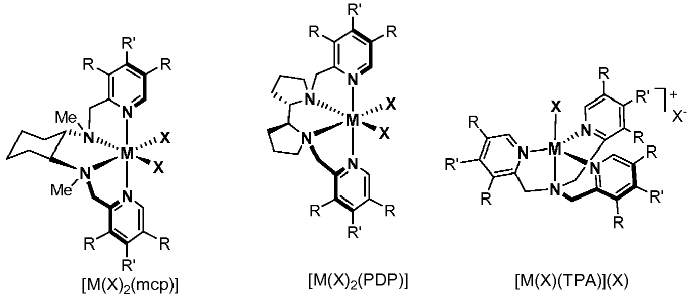 being M selected from Co and Ni and X being selected from chloride and trifluoromethanesulfonate and wherein R and R' are each a radical independently selected from the group consisting of hydrogen, (C₁-C₆)alkyl, (C₁-C₆)alkyloxy, and (C₁-C₆)alkyloxycarbonyl. Preferably, the cobalt (II) or nickel (II) compound is [Ni(OTf)(Py₂^{Ts}tacn)](OTf) or [Co(OTf)(Py₂^{Ts}tacn)](OTf). More preferably, it is [Ni(OTf)(Py₂^{Ts}tacn)](OTf).

In another particular embodiment of the third aspect of the invention, optionally in combination with one or more of the embodiments described below, the process comprises the step of contacting the compound comprising one or more moieties of formula C₁-Cl with the composition defined in any of the embodiments of the first aspect of the invention defined above under light irradiation and in the presence of an electron donor as defined above.

In another particular embodiment of the third aspect of the invention, optionally in combination with one or more of the embodiments described below, the process is carried out in the presence of a protic solvent, which is preferably a compound of formula R"OH wherein R" is selected from hydrogen and (C₁-C₆)alkyl. More preferably, the polar protic solvent is selected from the group consisting of methanol, ethanol and isopropanol. Such solvent may be used in combination with aprotic organic solvents such as acetonitrile, N,N-dimethylformamide, 1,4-dioxane, tetrahydrofuran, and acetone.

In a particular embodiment of the process of reduction of the invention, optionally in combination with one or more of the embodiments described above or below, the process is carried out at a temperature in a range from -10 °C to 50 °C; more preferably in the range from 20 °C to 40 °C. Even more preferably, optionally in combination with one or more of the embodiments described above or below, the process is carried out at a temperature of 30 °C.

In a preferred embodiment, optionally in combination with one or more of the embodiments described above or below, the molar ratio of the nickel (II) or cobalt (II) coordination complex to the compound comprising one or more moieties of formula C₁-Cl is in the range from 1:1000 to 1:10. Preferably, optionally in combination with one or more of the embodiments described above or below, the molar ratio of the nickel(II) or cobalt (II) coordination complex to the compound comprising one or more moieties of formula C₁-Cl is in the range from 1:100 to 1:10; and more preferably it is 5:100.

The following examples are provided by way of illustration, and they are not intended to be limiting of the present invention. Furthermore, the present invention covers all possible combinations of particular and preferred embodiments described herein.

### EXAMPLES

*Light source*: The reactions were performed using Royal-Blue (λ = 447±20 nm) LUXEON Rebel ES LED, mounted on a 20 mm Square Saber - 1030 mW @ 700mA as a light source or a Kessil® H150 LED system.

*Temperature Control:* Reaction temperature was controlled by a high precision thermoregulation Hubber K6 cryostat. Likewise, to guarantee a stable irradiation the temperature of the LEDs was also controlled and set up at 22 °C.

### Example 1: Process optimization

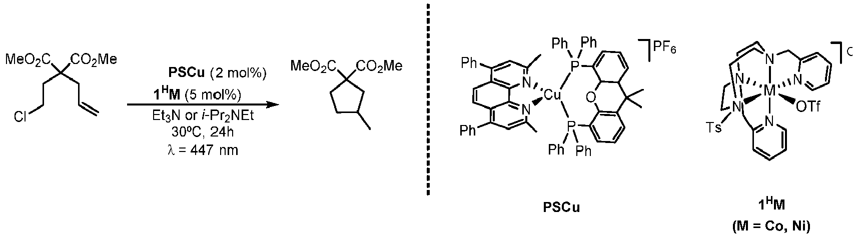

General Procedure 1: Inside an anaerobic box, aliquots from stock solutions of dimethyl allyl chloroethylmalonate (0.2 mL, 0.02 mmol, 1.0 eq.), the cobalt (II) or nickel (II) complex **1^{H}Co** or **1^{H}Ni** (0.1 mL, 1x10⁻³ mmol, 5 mol %), photosensitizer **PS**_{Cu} **(1)** (0.1 mL, 4x10⁻¹ mmol, 2 mol %) and acetonitrile (0.4 mL) were equally distributed into a vial (10 mL of headspace) that contained glass beads. The vial was sealed with a septum and removed from the anaerobic box. Degassed protic solvent was added to the vial to reach a total volume of 2 mL (total concentration of substrate 10 mM). Et₃N (40 µL, 0.286 mmol, 14.4 eq.) or *i*-Pr₂NEt (40 µL, 0.229 mmol, 11.4 eq.) was added to each vial, which was placed in the photoreactor at the indicated temperature (30 °C). After irradiating for 24h with blue LEDs (λ = 447 nm), the vial was opened and the mixture was diluted with ethyl acetate (2 mL). A solution of biphenyl in ethyl acetate was added as internal standard (8.7x10⁻³ mmols in 0.25 mL). Then, an aliquot of the organic phase, after extractions with 1 mL of H₂O, was passed through a plug of MgSO₄ and eluted with EtOAc. The resulting solution was analyzed by gas chromatography. The yields reported for each reaction are given as an average of two runs.

**Table 1**

| **Entry** | **Photosensitizer (% mol)** | **Catalyst (% mol)** | **Solvent (mL)** | **Electron donor** | **Yield (%)^{a}** |
|---|---|---|---|---|---|
| 1 | **PS_{Cu}** (5) | **1^{H}Co** (2) | MeCN | Et₃N (14.4) | 17 |
| 2 | **PS_{Cu}** (5) | **1^{H}Co** (2) | MeCN : H₂O (3:2) | Et₃N (14.4) | 22 |
| 3 | **PS_{Cu}** (5) | **1^{H}Co** (2) | MeCN : MeOH (3:2) | Et₃N (14.4) | 27 |
| 4 | **PS_{Cu}** (5) | **1^{H}Co** (2) | MeCN : *i*-PrOH (3:2) | Et₃N (14.4) | 61 |
| 5 | **PS_{Cu}** (5) | **1^{H}Co** (2) | MeCN : EtOH (3:2) | Et₃N (14.4) | 83 |
| 6 | **PS_{Cu}** (5) | **1^{H}Co** (2) | MeCN : EtOH (3:2) | *i*-Pr₂NEt (11.4) | 78 |
| 7 | **PS_{Cu}** (5) | Co(OTf)₂(MeCN)₂ | MeCN : EtOH (3:2) | Et₃N (14.4) | 5 |
| 8 | **PS_{Cu}** (5) | Co(OTf)₂(MeCN)₂ + dtbbpy^{b} | MeCN : EtOH (3:2) | Et₃N (14.4) | 10 |
| 9 | **PS_{Cu}** (5) | NiBr₂ · glyme + dtbbpy^{b} | MeCN : EtOH (3:2) | Et₃N (14.4) | 3 |
| 11 | **PS_{Ir1}** (5) | **1^{H}Co** (2) | MeCN : EtOH (3:2) | Et₃N (14.4) | 12 |
| 12 | **PS_{Ir2}** (5) | **1^{H}Co** (2) | MeCN : EtOH (3:2) | Et₃N (14.4) | 46 |
| 13 | **PS_{Cu}** (5) | **1^{H}Ni** (2) | MeCN : EtOH (3:2) | Et₃N (14.4) | 74 |
| 14 | **PS_{Cu}** (5) | **1^{H}Ni** (2) | MeCN : EtOH (3:2) | *i*-Pr₂NEt (11.4) | 96 |
| 15 | **PS_{Ir1}** (5) | **1^{H}Ni** (2) | MeCN : EtOH (3:2) | Et₃N (14.4) | 72 |
| 16 | **PS_{Ir2}** (5) | **1^{H}Ni** (2) | MeCN : EtOH (3:2) | Et₃N (14.4) | 73 |
| 17 | **PS_{Cu}** (5) | Ni(OTf)2(MeCN)2 | MeCN : EtOH (3:2) | Et₃N (14.4) | 1 |

| | | | | | |
|---|---|---|---|---|---|
| a) Yields were determined by GC using biphenyl as internal standard.b) dtbbpy = 4,4'-Di-*tert*-butyl-2,2'-dipyridyl. | | | | | |

Entries 7, 8, 9 and 17 fall out of the scope of the invention as they do not involve the use of tetradentate or pentadentate nitrogenated ligands and can be considered as comparative example.

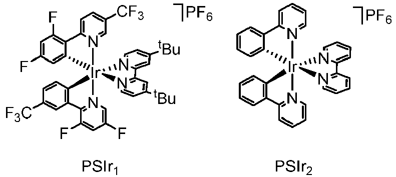

### Example 2: Screening of cobalt(II) or nickel (II) compound

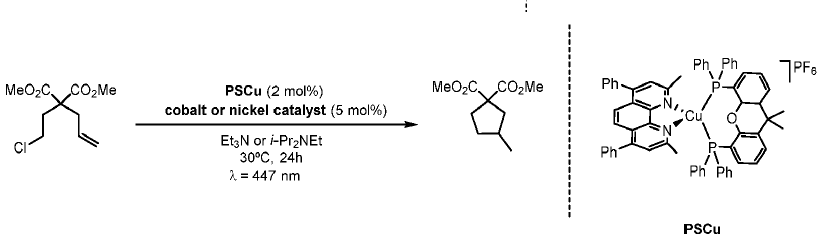

Following general procedure 1 but substituting the cobalt (II) or nickel (II) compound by the complex indicated in Table 2, the results obtained are indicated in Table 2.

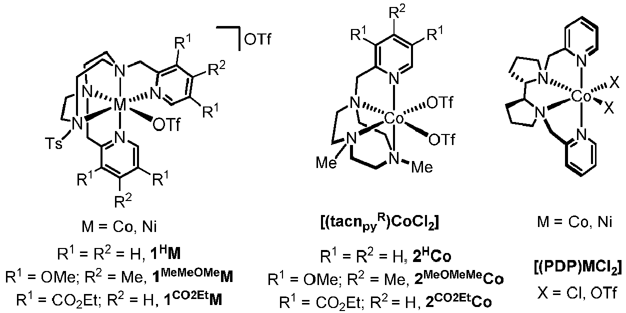

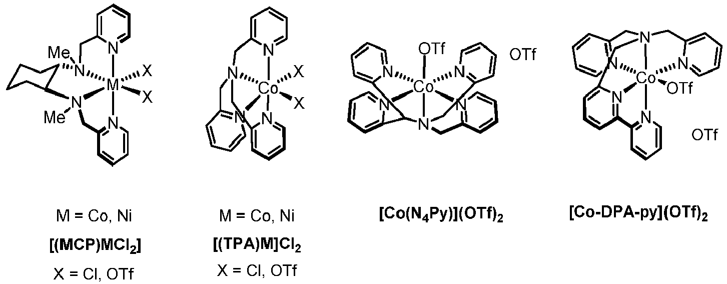

| Catalyst (amount in mol%) | Electron donor (equiv) | Conv. (%) | Yield (%) |
|---|---|---|---|
| 1^{H}Co (5) | Et₃N (14.3) | 96 | 83 |
| 1^{H}Ni (5) | *i*-Pr₂NEt (11.3) | 99 | 96 |
| 1^{MeOMeMe}Qo (5) | Et₃N (14.3) | 87 | 80 |
| 1^{MeOMeMe}Ni (5) | *i*-Pr₂NEt (11.3) | 70 | 52 |
| 1^{CO2Et}Co (5) | Et₃N (14.3) | 83 | 70 |
| 1^{CO2Et}Ni (5) | *i*-Pr₂NEt (11.3) | 73 | 63 |
| 2^{H}Co (5) | Et₃N (14.3) | 42 | 30 |
| 2^{MeOMeMe}Co (5) | Et₃N (14.3) | 22 | 18 |
| 2^{CO2Et}Co (5) | Et₃N (14.3) | 50 | 41 |
| Co-TPA_OTf₂ (5) | Et₃N (14.3) | 16 | 16 |
| Co-TPA_Cl₂ (5) | Et₃N (14.3) | 13 | 11 |
| Ni-TPA_OTf₂ | *i*-Pr₂NEt (11.3) | 96 | 54 |
| Co-MCP_OTf₂ (5) | Et₃N (14.3) | 71 | 68 |
| Co-MCP_Cl₂ (5) | Et₃N (14.3) | 71 | 64 |
| Ni-MCP_OTf₂ (5) | *i*-Pr₂NEt (11.3) | 90 | 55 |
| Co-PDP_OTf₂ (5) | Et₃N (14.3) | 67 | 60 |
| Ni-PDP_OTf₂ (5) | *i*-Pr₂NEt (11.3) | 94 | 68 |
| Co-DPA-BPy_OTf₂ (5) | Et₃N (14.3) | 97 | 89 |
| CoN₄Py_OTf₂ (5) | Et₃N (14.3) | 43 | 36 |

The results of Table 2 indicate that a broad range of tetradentate and pentadentate ligands are useful in the process of the invention.

### Example 3: Substrate scope

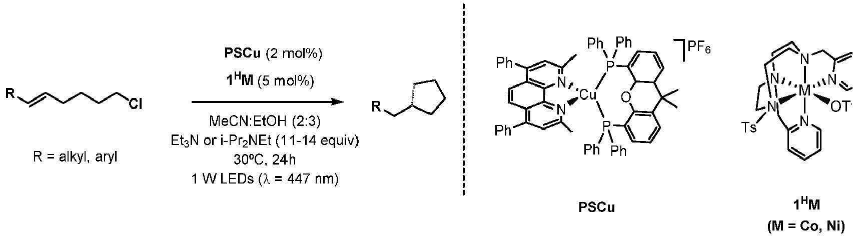

General procedure 2: Preparation in the Glovebox. A solution of chloroalkane starting material as shown in Table 3 (0.6 mL, 0.06 mmol, 1.0 eq.), complex **1^{H}Co** or **1^{H}Ni** as defined in Example 1 (0.6 mL, 0.003 mmol, 5 mol%), photosensitizer **PS**_{Cu} (0.6 mL, 0.0012 mmol, 3 mol%) and acetonitrile (0.6 mL) was equally distributed into Microwave-vials containing Glass Beads. The vials were sealed with a septum and removed from the Glovebox. Degased EtOH (three freeze-pump-thaw cycles) was added to each vial to reach a total volume of **6** mL (total concentration of substrate 10 mM). To these vials Et₃N (120 µL, 0.865 mmol, 14.4 eq.) or *i*-Pr₂NEt (120 µL, 0.687 mmol, 11.4 eq.) was added and the vials were placed in the photoreactor at the indicated temperature (30 °C) and stirring was switched on. The vials were irradiated for 24 h with visible light (Blue LED, 447 nm), opened and the content was combined in a separatory funnel. H₂O (15 mL) and Et₂O (15 mL) was added and the organic layer was separated. The aqueous layer was extracted with Et₂O (3 x 15 mL), and the combined organic extracts were washed with brine (15 mL) and dried over MgSO₄. The solvent was removed under reduced pressure and the crude material was purified via column chromatography.

**Table 3**

| Starting Material | Product | Catalyst | Isolated Yield (%) |
|---|---|---|---|
| 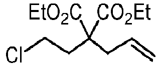 | 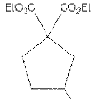 | 2^{a} | 88 |
| | | 3^{b} | 93 |
| 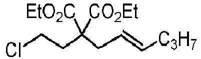 | 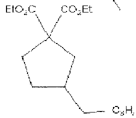 | 2^{a} | 50 |
| | | 3^{b} | 79 |
| 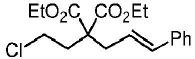 | 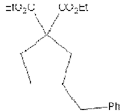 | 2^{a} | 73 |
| | | 3^{b} | 93 |
| 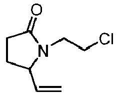 | 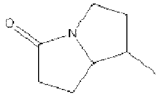 | 2^{a} | 83 |
| | | 3^{b} | 83 |
| 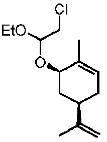 | 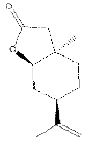 | 2^{a} | 62 |
| | | 3^{b} | 66 |
| 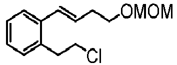 | 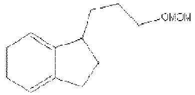 | 3^{b} | 52 |
| 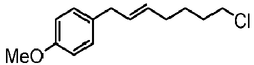 | 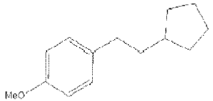 | 2^{a} | 37 |
| | | 3^{b} | 76 |
| 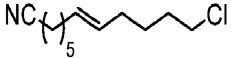 | 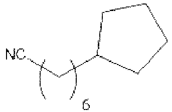 | 3^{b} | 52 |
| 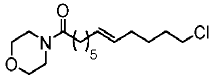 | 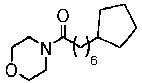 | 2^{a} | 23 |
| | | 3^{b} | 81 |
| 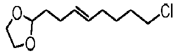 | 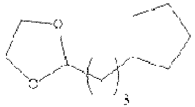 | 3^{b} | 40 |
| 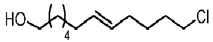 | 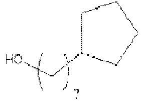 | 3^{b} | 77 |
| 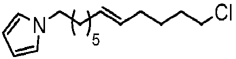 | 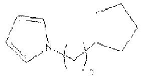 | 3^{b} | 48 |
| 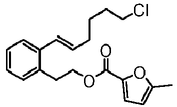 | 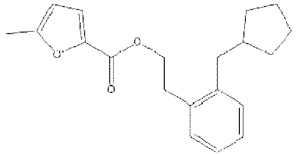 | 3^{b} | 88 |
| 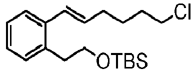 | 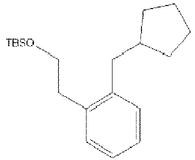 | 2^{a} | 23 |
| | | 3^{b} | 91 |
| 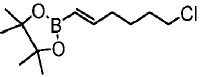 | 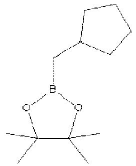 | 2^{a} | 14 |
| | | 3^{b} | 61 |
| 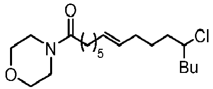 | 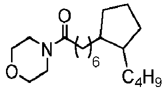 | 3^{b} | 67 |
| 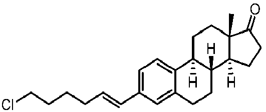 | 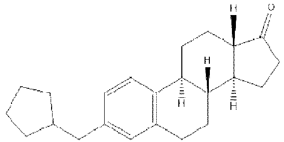 | 3^{b} | 79 |

| | | | |
|---|---|---|---|
| a: Triethylamine was used - Et₃N (120 µL, 0.865 mmol, 14.4 eq.), b) Diisopropylethylamine was used - *i*-Pr₂NEt (120 µL, 0.687 mmol, 11.4 eq.) | | | |

### Example 4: Boration of aryl chlorides

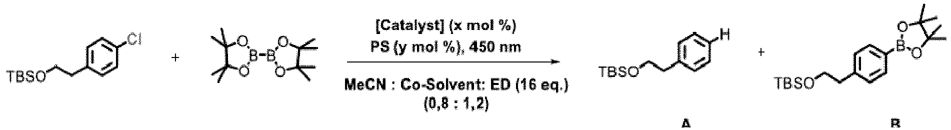

Inside an anaerobic box, aliquots from stock solutions of aryl chloride derivative (0.2 mL, 0.02 mmol, 1.0 eq.), the nickel (II) complex **1^{H}Ni** (0.1 mL, 5-10 mol %), photosensitizer **PS_{Cu}** (0.1 mL, 2-4 mol %) and acetonitrile (0.4 mL) were equally distributed into a vial (10 mL of headspace) that contained glass beads. The vial was sealed with a septum and removed from the anaerobic box. Degassed protic solvent was added to the vial to reach a total volume of 2 mL (total concentration of substrate 10 mM). Et₃N (40 µL, 14.4 eq.) or *i*-Pr₂NEt (40 µL, 11.4 eq.) was added to each vial, which was placed in the photoreactor at the indicated temperature (30 °C). After irradiating for 24h with blue LEDs (λ = 447 nm), the vial was opened and the mixture was diluted with ethyl acetate (2 mL). A solution of biphenyl in ethyl acetate was added as internal standard (8.7x10⁻³ mmols in 0.25 mL). Then, an aliquot of the organic phase, after extractions with 1 mL of H₂O, was passed through a plug of MgSO₄ and eluted with EtOAc. The resulting solution was analyzed by gas chromatography. The yields reported for each reaction are given as an average of two runs.

| Amount of B₂pin₂ (equiv) | Amount of **1^{H}Ni** (mol%) | Amount of **PS**_{Cu} (mol%) | Solvent | Yield A (%) | Yield B (%) |
|---|---|---|---|---|---|
| 2 | 10 | 4 | MeCN : EtOH (0.8:1.2) | 68 | 11 |
| 4 | 10 | 4 | MeCN : EtOH (0.8:1.2) | 38 | 21 |
| 4 | 10 | 4 | MeCN : EtOH 1:0.2) | 14 | 17 |
| 4 | 5 | 2 | MeCN : EtOH (1:0.2) | 11 | 11 |

### REFERENCES CITED IN THE APPLICATION

1. Nature Reviews Chemistry 2017, 1, article 52
2. Angew. Chem. Int. Ed. 2013, 52, 13342 -13345
3. Science 2014, 346, 725-728
4. Angew. Chem. Int. Ed. 2012, 51, 12303-12306
5. ACS Catalysis, 2017, 7, 409-412
6. Nature Chemistry 2012, 4, 854-859
7. Chem.Comm. 2010, 46, 4985-4987

## Claims

1. A composition comprising a catalyst system consisting of: a compound of formula [Cu(L₁)(L₂)]Z₁, wherein:
Cu is in the oxidation state (+1);
Z₁ is an anion
L₁ is a bidentate phosphine ligand attached to the Cu atom through the P atoms
L₂ is a bidentate nitrogen ligand attached to the Cu atom through two N atom consisting of a ring system comprising from 2 to 7 rings, wherein: the rings have from 5 to 6 members selected from the group consisting of C, CH, N, and NH; at least two members being N or NH;
the N atoms attached to the copper atom are separated by no more than three ring members and are members of different rings;
the rings are unsaturated or aromatic;
the rings are fused or interconnected;
the rings are optionally substituted with one or more radicals selected from the group consisting of: (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro;
and a cobalt (II) or nickel(II) compound selected from the compounds of formula [Co(L₃)X]⁺X'⁻, [Co(L₃)XX'], [Ni(L₃)X]⁺X'⁻and [Ni(L₃)XX'] wherein:
X and X' are anions equal or different;
L₃ is a tetradentate or pentadentate nitrogenated ligand attached to the Co or Ni atom through four or five N atoms that is selected from the compounds of formula (Ia), (Ib), (Ic), (Id) and (Ie) 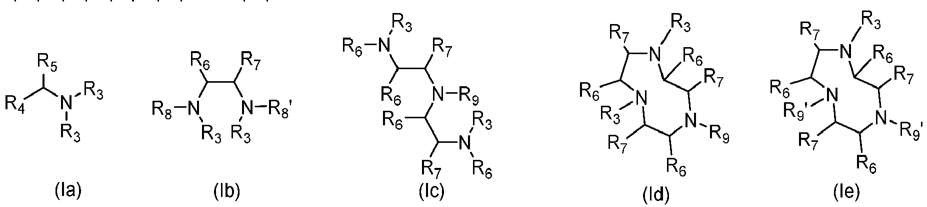
wherein each R₃ is a radical of formula (II) 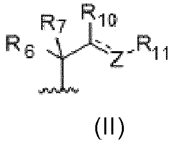
wherein Z is N or NH;
the dashed bond represents a single C-Z bond or a double C=Z bond; and R₁₀ and
R₁₁, together with the atoms to which they are attached, form a ring system comprising from one to three unsaturated or aromatic 5 to 6-membered rings, said rings being fused or interconnected, the members of the ring being selected from C, CH, N and NH, the rings being further optionally susbtituted with one or more radicals selected from the group consisting of (C₁-C₆)alkyl, (C₁-C₆)haloalkyl, halo, (C₁-C₆)alkyloxy, (C₁-C₆)alkyloxycarbonyl, (C₁-C₆)alkylcarbonyloxy, cyano and nitro;
R₄ and R₅ are each independently selected from the group consisting of: hydrogen, (C₁-C₆)alkyl, (C₁-C₆)haloalkyl, and a radical of formula -C(R₁₀)(ZR₁₁); being at least one of R₄ and R₅ a radical of formula -C(R₁₀)(ZR₁₁);
R₆ and R₇ are each independently selected from the group consisting of: hydrogen, (C₁-C₆)alkyl, halo, (C₁-C₆)haloalkyl, and (C₁-C₆)alkyloxy; or, alternatively,
R₆ and R₇, together with the carbon atoms to which they are attached form a (C₃-C₈)cycloalkyl optionally substituted with one or more radicals selected from the group consisting of: (C₁-C₆)alkyl, halo, (C₁-C₆)haloalkyl, and (C₁-C₆)alkyloxy;
R₈ is a radical selected from the group consisting of hydrogen, (C₁-C₆)alkyl, and (C₁-C₆)haloalkyl;
R_{8'} is a radical selected from the group consisting of hydrogen, (C₁-C₆)alkyl, (C₁-C₆)haloalkyl and a radical of formula (II);
or, alternatively,
one or two of the pairs of R₆ and R₈ and R₇ and R₈' together with the carbon atoms to which they are attached form a (C₃-C₈)Cycloalkyl optionally substituted with one or more radicals selected from the group consisting of (C₁-C₆)alkyl, halo, (C₁-C₆)haloalkyl, and (C₁-C₆)alkyloxy;
Rg and Rg' are each independently selected from the group consisting of hydrogen, (C₁-C₆)alkyl, a radical of formula R₁₂O(C=O)-, R₁₂(C=O)-, R₁₂(S=O)- and R₁₂(SO₂)-wherein R₁₂ is selected from the group consisting of (C₁-C₆)alkyl, (C₁-C₆)haloalkyl,
benzyl and phenyl optionally substituted with (C₁-C₆)alkyl and (C₁-C₆)haloalkyl; and wherein:
when the cobalt (II) compound is a compound of formula [Co(L₃)X]⁺X'⁻, L₃ is a pentadentate nitrogenated ligand; and
when the cobalt (II) compound is a compound of formula [Co(L₃)XX'], L₃ is a tetradentate nitrogenated ligand;
when the nickel (II) compound is a compound of formula [Ni(L₃)X]⁺X'⁻, L₃ is a pentadentate nitrogenated ligand; and
when the nickel (II) compound is a compound of formula [Ni(L₃)XX'], L₃ is a tetradentate nitrogenated ligand.

2. The composition of claim 1 wherein the cobalt (II) or nickel (II) compound is selected from: [M(X)(Py₂^{Ts}tacn)](X), [M(X)(Py₂^{Me}tacn)](X), [M(X)(DPA-Bpy)](X), [M(X)(N4Py)](X), [M(X)₂(PDP)], [M(X)(TPA)](X), and [M(X)₂(mcp)] 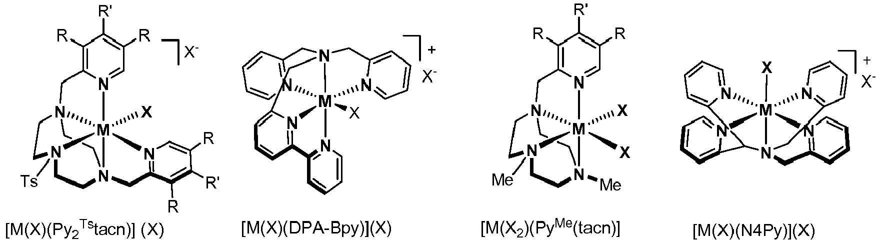 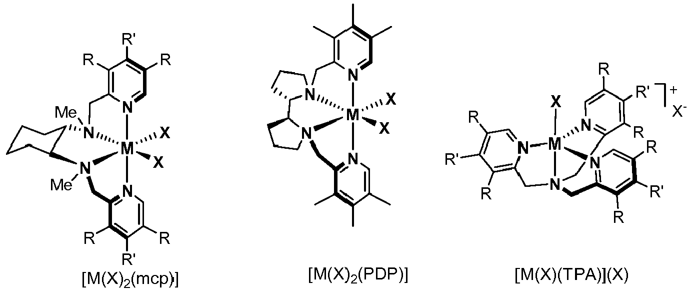
being M selected from Co and Ni and X being selected from chloride and trifluoromethanesulfonate
and wherein R and R' are each a radical independently selected from the group consisting of hydrogen, (C₁-C₆)alkyl, (C₁-C₆)alkyloxy, and (C₁-C₆)alkyloxycarbonyl.

3. The composition according to any of the claims 1 and 2 wherein the [Cu(L₁)(L₂)]Z₁ is [Cu(bathocuproine)(xantphos)](PF₆)

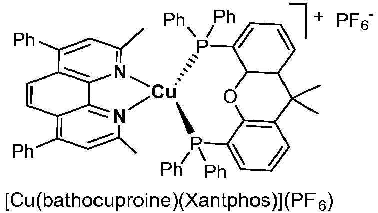

4. The composition according to any of the claims 2 to 3 wherein the cobalt (II) or nickel (II) compound is [Co(OTf)(Py₂^{Ts}tacn)](OTf) or [Ni(OTf)(Py₂^{Ts}tacn)](OTf); preferably it is [Ni(OTf)(Py₂^{Ts}tacn)](OTf).

5. The composition according to any of the claims 1 to 4 wherein the molar ratio of the compound of formula [Cu(L₁)(L₂)]Z₁ to the cobalt (II) or nickel (II) compound is comprised from 0.2:1 to 1:1; preferably it is 0.4:1.

6. Use of the composition of any of the claims 1 to 5 as a catalyst in combination with an electron donor and a source of light.

7. Use according to claim 6 wherein the electron donor is a compound of formula NR₂₃R₂₄R₂₅ wherein each of R₂₃, R₂₄ and R₂₅ is independently a (C₁-C₆)alkyl.

8. Use according to any of claims 6 and 7 wherein, when the compound of formula [Cu(L₁)(L₂)]Z₁ is [Cu(bathocuproine)(xantphos)](PF₆), the source of light has a wavelength comprised from 400 to 500 nm.

9. A process for the preparation of a compound comprising one or more moieties of formula C₁-Y from a compound comprising one or more moieties of formula C₁-Cl, the moieties of formula C₁-Cl being converted in the moieties of formula C₁-Y, wherein C₁ is a saturated carbon atom or a carbon atom comprised in a carbon-carbon double bond and Y is an atom selected from C₂, H and B, being C₂ a saturated carbon atom, said process comprising the step of contacting the compound comprising one or more moieties of formula C₁-Cl with a catalytically effective amount of a cobalt(ll) or nickel (II) coordination complex with a tetradentate or pentadentate nitrogenated ligand in the presence of a reducing agent and wherein:
when Y is C₂, the moiety of formula C₁-Cl is comprised in a moiety of formula (III) and the moiety of formula C₁-C₂ is comprised in a moiety of formula (IV) 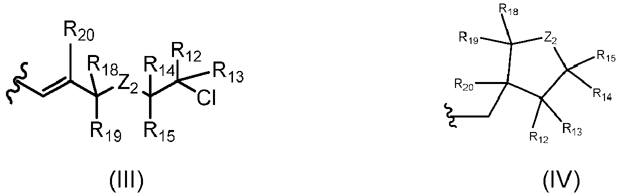
wherein Z₂ is selected from NR₁₆, CR₁₆R₁₇, S, PR₁₆ and O,
each of R₁₂, R₁₃, R₁₄, R₁₅, R₁₆, R₁₇, R₁₈, R₁₉ and R₂₀ is independently selected from the group consisting of hydrogen, (C₁-C₆)alkyl, (C₂-C₆)alkenyl, (C₂-C₆)alkynyl, (C₁-C₆)haloalkyl, (C₁-C₆)alkyloxy, (C₁-C₆)alkylamino, (C₁-C₆)alkylcarbonyl, (C₁-C₆)alkylcarbonyloxy, (C₁-C₆)alkyloxycarbonyl, (C₁-C₆)alkylcarbonylamino, (C₁-C₆)alkylaminocarbonyl, cyano and nitro; or
alternatively, one or two of the pairs of R₁₃ and R₁₄, R₁₄ and R₁₆, R₁₆ and R₁₉ and R₁₉ and R₂₀, together with the carbon atoms to which they are attached, form a ring system comprising from 1 to 3 saturated, unsaturated or aromatic rings,
where chemically possible, each ring comprising from 3 to 8 members selected from the group consisting of C, CH, CH₂, O, N, CO, CS and S, the rings being further optionally substituted at any available position with one or more groups selected from the group consisting of (C₁-C₆)alkyl, (C₂-C₆)alkenyl, (C₂-C₆)alkynyl, (C₁-C₆)haloalkyl, (C₁-C₆)alkyloxy, (C₁-C₆)alkylamino, (C₁-C₆)alkylcarbonyl, (C₁-C₆)alkylcarbonyloxy, (C₁-C₆)alkyloxycarbonyl, (C₁-C₆)alkylcarbonylamino, (C₁-C₆)alkylaminocarbonyl, cyano and nitro;
R₁₆ and R₁₇ are each independently selected from the group consisting of hydrogen, (C₁-C₆)alkyl, (C₂-C₆)alkenyl, (C₂-C₆)alkynyl, (C₁-C₆)haloalkyl, (and (C₁-C6)alkylcarbonyl; or, alternatively, the moieties of formula CR₁₄R₁₅ and CR₁₆R₁₇, CR₁₈R₁₉ further optionally form a carbonyl group;
or, alternatively,
when Y is B, C₁ is comprised in a carbon-carbon double bond and the process is further carried out and in the presence of a compound of formula [B(OR₂₁)(OR₂₂)]₂ wherein each of R₂₁ and R₂₂ is independently selected from (C₁-C₆)alkyl, (C₂-C₆)alkenyl, (C₂-C₆)alkynyl, and (C₁-C₆)haloalkyl; or, alternatively, R₂₁ and R₂₂, together with the oxygen atoms to which they are attached, said oxygen atoms being bridged by a boron atom, form a 5 to 6-membered saturated carbocyclic ring optionally substituted at any available position with one or more (C₁-C₆)alkyl groups.

10. The process of claim 9 wherein the reducing agent is able to reduce the cobalt (II) or nickel (II) compound to a cobalt (I) or nickel (I) compound and is selected from the group consisting of photochemical reducing agents, electrochemical reducing agents and chemical reducing agents, and wherein when the reducing agent is a photochemical reducing agent, the process is further carried out under light irradiation.

11. The process of claim 10 wherein the reducing agent is a photochemical reducing agent consisting of the combination of a photosensitizer and an electron donor.

12. The process of claim 11 wherein the photosensitizer is selected from the group consisting of hexacoordinated ruthenium(II) complexes with polypyridyl ligands, hexacoordinated iridium(III) complexes with pyridyl and/or polypyridyl ligands, and a compound of formula [Cu(L₁)(L₂)]Z₁ as defined in any of the claims 1 to 5 and the electron donor is selected form the group consisting of a compound of formula NR₂₃R₂₄R₂₅ wherein each of R₂₃, R₂₄ and R₂₅ is independently a (C₁-C₆)alkyl, methanol, formaldehyde, formic acid, oxalic acid, ascorbic acid, lactic acid and an electrochemical half-cell.

13. The process according to claim 12 wherein the photosensitizer is a compound of formula [Cu(L₁)(L₂)]Z₁ as defined in any of the claims 1 to 5, the electron donor is a compound of formula NR₂₃R₂₄R₂₅ wherein each of R₂₃, R₂₄ and R₂₅ is independently a (C₁-C₆)alkyl and the cobalt (II) or nickel (II) compound is as defined in any of the claims 1 to 5.

14. The process according to claim 13 wherein the photosensitizer is a compound of formula [Cu(L₁)(L₂)]Z₁ as defined in claim 3, the electron donor is selected from the group consisting of trimethylamine and diisopropylethylamine and the cobalt (II) or nickel (II) compound is as defined in any of the claims 2 and 4.

15. The process according to claim 9 which comprises the step of contacting the compound comprising one or more moieties of formula C₁-Cl with the composition defined in any of the claims 1 to 5 under light irradiation and in the presence of an electron donor as defined in any of the claims 12 to 14.
